Europäisches Patentamt

(19) European Patent Office

Office européen des brevets

(11) Numéro de publication : **0 300 895 B1**

(12) **FASCICULE DE BREVET EUROPEEN**

(45) Date de publication du fascicule du brevet :
04.09.91 Bulletin 91/36

(21) Numéro de dépôt : **88401867.2**

(22) Date de dépôt : **20.07.88**

(51) Int. Cl.$^5$ : **A23L 1/30, A23L 1/303, A23L 1/304**

(54) **Mélange nutritif destiné à la personne âgée.**

(30) Priorité : 23.07.87 FR 8710405

(43) Date de publication de la demande :
25.01.89 Bulletin 89/04

(45) Mention de la délivrance du brevet :
04.09.91 Bulletin 91/36

(84) Etats contractants désignés :
**AT BE CH DE FR GB IT LI LU NL SE**

(56) Documents cités :
**EP-A- 0 102 663**
**WO-A-86/05660**
**FR-A- 2 154 397**

(56) Documents cités :
CHEMICAL ABSTRACTS, vol. 104, no. 19, ma 1986, page 561, résumé no. 167217d, Columbus, Ohio, US; & JP-A-61 15 648 (ASAHI DENKA KOGYO K.K.) 23-01-1986
CHEMICAL ABSTRACTS, vol. 102, no. 23, juir 1985, page 511, résumé no. 202916p, Columbus, Ohio, US; && JP-A-60 02 154 (SANYU SHOJI CO., LTD) 08-01-1985

(73) Titulaire : SYNTHELABO
58 rue de la Glacière
F-75013 Paris (FR)

(72) Inventeur : Darmenton, Patrick
6, rue Auboin
F-92340 Bourg la Reine (FR)

(74) Mandataire : Thouret-Lemaitre, Elisabeth et al
SYNTHELABO Service Brevets 22, avenue Galilée
F-92352 LE PLESSIS ROBINSON CEDEX (FR)

## Description

La présente invention a pour objet un mélange nutritif destiné à la personne agée.

Cinquante à soixante pour cent des vieillards hospitalisés sont dénutris (A. ROMAGNOLI — JULLARD et coll. — 1984 ; A. DEVILLE CHABROLE — 1986) et leur apport lipidique est très souvent déséquilibré.

L'alitement prolongé des vieillards conduit à une morbidité accrue.

Par ailleurs le vieillissement de la population est un phénomène bien connu dans les pays européens et aux Etats-Unis.

Le vieillissement entraîne d'importants changements physiologiques susceptibles d'affecter les besoins en certains nutriments, vitamines ou minéraux.

A l'heure actuelle il n'existe aucun produit spécifique complet pour le vieillard.

Le mélange nutritif de l'invention est une préparation complète, polymérique, prête à l'emploi, sous forme d'une émulsion fine d'une granulométrie comprise entre 300 et 400 nm. Administré par sonde ou par voie orale, il est spécifiquement adapté aux besoins nutritionnels de la personne âgée,

— puisqu'il tient compte des besoins en protides, lipides, vitamines, minéraux et oligoéléments.. propres à la personne âgée et de l'absorption intestinale,

— et qu'il permet de nourrir le vieillard en prévenant les complications dues au stress.

Le choix des constituants du mélange nutritif de l'invention vise plusieurs objectifs.

1. Equilibrer la balance azotée.

La balance azotée est déficitaire chez le sujet agé ; ce déficit est aggravé par une situation de stress. L'apport calorico-azoté doit être suffisant pour assurer une évolution favorable.

2. Faciliter les phénomènes de cicatrisation.

La restauration d'un bon apport protéique en pré et post-opératoire, favorise la cicatrisation.

Cependant la carence en folates entraîne un retard à la cicatrisation.

Le mélange nutritif de l'invention comporte des protéines en quantité allant de 10 à 16 g par jour et de l'acide folique selon un apport journalier compris entre 2 et 7 mg.

Par ailleurs il s'est avéré utile dans certains cas que, parmi les protéines, il y ait de l'arginine.

3. Assurer un bon apport lipidique en particulier en acides linoléique et eicosapentaénoïque.

A cet effet le mélange nutritif de l'invention contient un mélange de 2 ou 3 huiles végétales et d'une huile de poisson, mélange qui apporte les quantités requises en acides gras. L'apport lipidique de l'émulsion de l'invention est caractérisé par un rapport entre la somme des acides gras essentiels et la sommes des acides gras saturés allant de 0,4 à 1,0 et par un rapport entre la somme des acides gras en $n - 6$ et la somme des acide gras en $n - 3$ allant de 3,5 à 10,0, de manière à fournir des quantités importantes d'acides linoléique et eicosapentaénoïque.

Le mélange nutritif de l'invention contient également des TCM (triglycérides à chaîne moyenne) (20 à 30% de l'apport lipidique) pour fournir à la personne agée une source lipidique très rapidement absorbable par voie intestinale et dont la pénétration dans les mitochondries ne nécessite pas de carnitine.

4. Normaliser la tolérance au glucose.

La personne agée réagit plus lentement à la surcharge glucidique comme le test d'hyperglycémie provoqué peut le révéler. Aussi, le mélange nutritif de l'invention contient du chrome (150 à 250 µg) qui potentialise l'action de l'insuline en périphérie, et de la niacine (30-120 mg) qui renforce l'action de l'oligoélément ajouté.

5. Accroître les défenses immunitaires.

Parmi les différents facteurs qui agissent sur les défenses immunitaires, le zinc joue un rôle primordial. Le mélange nutritif de l'invention contient de 2 à 4 mg de zinc pour 100 ml d'émulsion.

Il contient également d'autres constituants qui interviennent à divers degrés : vitamine A (apport journalier: 4000 à 6000 UI), vitamine $B_6$ qui affecte l'immunité cellulaire (apport journalier : 10 à 20 mg).

6. Lutter contre la décalcification.

Les pertes en calcium, d'origine hormonale, qui affectent la personne âgée sont responsables de 50.000 fractures par an et de 1.000.000 de tassements vertébraux, principalement chez la femme.

Le mélange nutritif de l'invention contient
— du calcium à un taux supérieur à 1g/jour,
— de la vitamine D à un taux compris entre 1000 et 2200 UI/jour,
— du fluor à un taux compris entre 1 et 5 mg/jour.

Par ailleurs, les TCM présents dans l'apport lipidique au taux de 20 à 30%, favoriseraient l'absorption du calcium, des acides aminés et du magnésium (A.C. BACH — V.K. BABAYAN — 1982).

7. Diminuer les phénomènes d'oxydation.

Parmi les causes du vieillissement, une des hypothèses évoquées est celle "des radicaux libres". Il a été démontré que le seuil de survie s'élève chez des animaux dont la nourriture a été enrichie en antioxydants.

De plus, l'état de stress du patient se caractérise par l'apparition de radicaux libres dont la présence n'est pas souhaitable, surtout si le patient est alimenté avec des lipides insaturés (KIYOSHI TAKEDA et coll. — 1984 — 1986).

Le mélange nutritif de l'invention contient
— un antioxydant liposoluble : la vitamine E(30 à 150 mg/jour)
— un antioxydant hydrosoluble : la vitamine C (150 à 300 mg/jour)
— du sélénium (40-150 μg/jour). Cet oligoélément est le cofacteur de la glutathion-oxydase dont le rôle est de réduire les peroxydes (J.L. ROMBEAU — 1984).

8. Faciliter le transit intestinal.

Le vieillard présente très souvent "une paresse intestinal" qu'il faut combattre.

La présence de fibres dans l'émulsion y contribue, elle est souhaitable sous forme d'un apport journalier de 3 à 12 grammes.

Les exemples suivants illustrent l'invention.

Exemple 1 :                                          Apport journalier

A Protéines :                                              84 à 97 g

B Lipides :                                                68 à 76 g
composition : TCM              20   %
huile de bourrache            7,7 %
huile d'olive européenne   45,0 %
huile de soja                19,5 %
huile de foie de morue        7,8 %

C Glucides :                                             240 à 265 g
Hydrolysat de polyoses renfermant encore
60 à 80 % de polysaccharides.

D . Vitamines :

| | | |
|---|---|---|
| A (UI) | 4000 à 6000 |
| $B_1$ (mg) | 3 à 5 |
| $B_2$ (mg) | 3 à 6 |
| $B_6$ (mg) | 10 à 20 |
| $B_{12}$ (µg) | 5 à 25 |
| C (mg) | 150 à 300 |
| D (UI) | 1200 à 2200 |
| E (mg) | 30 à 150 |
| NIACINE (mg) | 30 à 120 |
| Acide folique (mg) | 2 à 6 |
| Acide panthoténique (mg) | 10 à 35 |
| BIOTINE (µg) | 180 à 260 |

E . Minéraux :

| | |
|---|---|
| Na (mg) | 1875 à 2250 |
| K (mg) | 900 à 2250 |
| Ca (mg) | 1200 à 3000 |
| P (mg) | 1000 à 2000 |
| Mg (mg) | 450 à 900 |
| Fe (mg) | 16 à 30 |
| Zn (mg) | 40 à 60 |
| Cl (mg) | 1000 à 5100 |

F . Oligoéléments :

| | |
|---|---|
| I (µg) | 120 à 200 |
| Mn (mg) | 2,5 à 5 |
| Cu (mg) | 2,5 à 5 |
| Cr (µg) | 150 à 250 |
| Mo (µg) | 275 à 350 |
| F (mg) | 1 à 5 |
| Se (µg) | 40 à 150 |

G Divers
- L-carnitine (mg)                                    200 à 500
- émulsionnant : lécithine de soja (g)               1,33 - 26,6
- fibres (de betterave à sucre) (g)
  (éventuellement)                                        3 - 12
- Arôme (tels que vanille, moka, fraise ou
  chocolat)
- Solution de NaOH ou HCl pour ajuster le pH
- Eau (ml) qsp                                            2000

$\underline{\Sigma AG\ ESSENTIELS} = 0,74 \qquad \underline{\Sigma AG\ (n-6)} = 8,8$
$\Sigma AG\ SATURES \qquad\qquad\qquad \Sigma AG\ (n-3)$

Exemple 2

A - C - D - E identiques à l'exemple 1

B Lipides :

Composition : TCM                20    %
huile de bourrache               15,3  %
huile d'olive européenne         45,2  %
huile de noix                    15,1  %
huile de sardine                  4,4  %

$\underline{\Sigma AG\ ESSENTIELS} = 0,76 \qquad \underline{\Sigma AG\ (n-6)} = 5,30$
$\Sigma AG\ SATURES \qquad\qquad\qquad \Sigma AG\ (n-3)$

Exemple 3

A - C - D - E identiques à l'exemple 1

B Lipides :

Composition : TCM                20    %
huile de bourrache                4    %
huile d'olive européenne         56,5  %
huile de soja                    11,8  %
huile de foie de morue            7,7  %

$\underline{\Sigma AG\ ESSENTIELS} = 0,53 \qquad \underline{\Sigma AG\ (n-6)} = 5,5$
$\Sigma AG\ SATURES \qquad\qquad\qquad \Sigma AG\ (n-3)$

Exemple 4 :
A - C - D - E identiques à l'exemple 1
B Lipides :                                              68 à 76 g
   Composition : TCM              20    %
   huile de bourrache            15,3 %
   huile d'olive européenne 42,8 %
   huile de noix                14,0 %
   huile de foie de morue        7,9 %

$\underline{\Sigma AG\ ESSENTIELS} = 0,75$        $\underline{\Sigma AG\ (n-6)} = 4,83$
$\Sigma AG\ SATURES$                              $\Sigma AG\ (n-3)$


Exemple 5 :
A - C - D - E identiques à l'exemple 1
B Lipides :
   composition : TCM             20     %
   huile de bourrache            15,3   %
   huile d'olive européenne 40,31 %
   huile de noix                13,0   %
   huile de hareng              11,4   %

$\underline{\Sigma AG\ ESSENTIELS} = 0,71$        $\underline{\Sigma AG\ (n-6)} = 5,07$
$\Sigma AG\ SATURES$                              $\Sigma AG\ (n-3)$


Exemple 6 :
A - C - D - E identiques à l'exemple 1
B Lipides :                                              68 à 76 g
   composition : TCM                            20     %
                huile de bourrache            15,3 %
                huile d'olive européenne 34,6 %
                huile de soja                18,7 %
                huile de hareng              11,4 %

$\underline{\Sigma AG\ ESSENTIELS} = 0,74$        $\underline{\Sigma AG\ (n-6)} = 6,50$
$\Sigma AG\ SATURES$                              $\Sigma AG,\ (n-3)$

Exemple 7 :

A - C - D - E identique à l'exemple 1

B Lipides :             68 à 76 g

 composition : TCM    30   %

 huile de bourrache   15,3 %

 huile de foie de morue 2,4 %

 huile de colza    47,2 %

$\dfrac{\Sigma\text{AG ESSENTIELS}}{\Sigma\text{AG SATURES}} = 0,67$    $\dfrac{\Sigma\text{AG (n-6)}}{\Sigma\text{AG, (n-3)}} = 4,76$

Exemple 8 :

A - C - D - E identiques à l'exemple 1

B Lipides :             68 à 76 g

 composition : TCM     30   %

 huile de bourrache    20,4 %

 huile de hareng     3,5 %

 huile de lupin (1) ou de colza(2) 46,1 %

(1) $\dfrac{\Sigma\text{AG ESSENTIELS}}{\Sigma\text{AG SATURES}} = 0,56$    $\dfrac{\Sigma\text{AG (n-6)}}{\Sigma\text{AG (n-3)}} = 4,53$

(2) $\dfrac{\Sigma\text{AG ESSENTIELS}}{\Sigma\text{AG SATURES}} = 0,66$    $\dfrac{\Sigma\text{AG (n-6)}}{\Sigma\text{AG (n-3)}} = 4,81$

Exemple 9 :

A - C - D - E identiques à l'exemple 1

B Lipides :             68 à 76 g

 composition : TCM          30   %

        huile de bourrache      20,4 %

        huile de sardine       1,4 %

        huile de lupin (1) ou de colza (2) 48,2 %

(1) $\dfrac{\Sigma\text{AG ESSENTIELS}}{\Sigma\text{AG SATURES}} = 0,57$    $\dfrac{\Sigma\text{AG (n-6)}}{\Sigma\text{AG (n-3)}} = 4,42$

(2) $\dfrac{\Sigma\text{AG ESSENTIELS}}{\Sigma\text{AG SATURES}} = 0,67$    $\dfrac{\Sigma\text{AG (n-6)}}{\Sigma\text{AG (n-3)}} = 4,70$

Exemple 10 :

A - C - D - E identiques à l'exemple 1

B Lipides :                                                    68 à 76

  composition : TCM                                    30    %

               huile de cassis                    17,6 %

               huile de hareng                     3,5 %

               huile d'arachide           (1)   )

          ou huile de carthame           (2)   (   48,9 %

          ou huile d'olive européenne    (3)   )

          ou huile de palme d'amérique   (4)   (

$\Sigma$AG ESSENTIELS = (1) 0,59    $\Sigma$AG (n-6) = (1) 6,75

$\Sigma$AG SATURES      (2) 0,68    $\Sigma$AG (n-3)  (2) 6,67

             (3) 0,46        (3) 4,22

             (4) 0,56        (4) 5,69

Exemple 11 :

A - C - D - E identiques à l'exemple 1

B Lipides :                                                    68 à 76 g

  composition : TCM                                    30    %

               huile de cassis                    17,6 %

               huile de foie de morue              2,4 %

               huile de carthame          (1)   )

          ou huile de noisette           (2)   (   49,9 %

          ou huile d'olive               (3)   )

          ou huile de palme d'amérique   (4)   (

$\Sigma$AG ESSENTIELS = (1) 0,69    $\Sigma$AG (n-6) = (1) 6,76

$\Sigma$AG SATURES      (2) 0,64    $\Sigma$AG (n-3)  (2) 5,95

             (3) 0,53        (3) 5,33

             (4) 0,57        (4) 5,74

Exemple 12 :

A - C - D - E identiques l'exemple 1

B Lipides :                                                    68 à 76 g

   composition : TCM                                    30 %

                  huile de cassis                    17,6 %

                  huile de sardine                    1,4 %

                  huile de carthame            (1) )

         ou huile d'olive d'afrique   (2) (   50,9 %

         ou huile de palme d'amérique (3) )

$\Sigma$AG ESSENTIELS = (1) 0,70     $\Sigma$AG (n-6) = (1) 6,81

$\Sigma$AG SATURES       (2) 0,53     $\Sigma$AG (n-3)   (2) 4,76

                (3) 0,57                     (3) 5,77

## Revendications

1. Mélange nutritif complet, polymérique, sous forme d'émulsion fine, destiné à la nutrition par voie orale ou entérale des personnes âgées, mélange contenant des protéines, des lipides, des glucides, des vitamines, des minéraux et des oligoéléments, mélange caractérisé en ce qu'il contient
   - du calcium (> 0,066 g/100 ml) associé à du fluor (0,05 à 0,25 mg/100 ml) et de la vitamine D (60 à 110 UI/100 ml),
   - de l'acide folique (0,13 à à 0,4 mg/100 ml), et
   - du zinc (2 à 4,0 mg/100 ml).

2. Mélange nutritif selon la revendication 1, caractérisé par le fait que l'apport lipidique
   1) contient de l'acide eicosapentaénoïque C20 :5, (n – 3) (0,25 à 0,75% de l'apport lipidique) et
   2) répond aux critères suivants

$$\frac{\Sigma\text{AG Acides gras essentiels}}{\Sigma\text{AG Acides gras saturés}} = 0,4 \text{ à } 1,0$$

$$\frac{\Sigma\text{AG Acides gras série (n – 6)}}{\Sigma\text{AG Acide gras série (n – 3)}} = 3,5 \text{ à } 10,0$$

3. Mélange nutritif selon l'une quelconque des revendications 1 et 2, caractérisé en ce qu'il contient du chrome sous la forme d'association Niacine (2-8 mg/100 ml) et chrome (10 à 16,7 µg/100 ml).

4. Mélange nutritif selon l'une quelconque des revendications 1 à 3, caractérisé par le fait qu'il contient une source de protéines enrichie en arginine (0,66 g à 1,07 g/100 ml).

## Patentansprüche

1. Vollständiges Nahrungsgemisch der Kategorie "polymerisch" in Form einer feinen Emulsion zur Ernährung von alten Menschen auf oralem oder enteralem Weg, welches Gemisch Proteine, Lipide, Zucker, Vitamine, Mineralstoffe und Oligoelemente enthält und dadurch gekennzeichnet ist, daß es enthält :
   — Kalzium (> 0,066 g/100 ml) in Kombination mit Fluor (0,05 bis 0,25 mg/100 ml) und Vitamin D (60 bis 110 IE/100 ml),
   — Folsäure (0,13 bis 0,4 ml/100 ml) und
   — Zink (2 bis 4,0 mg/100 ml)

2. Nahrungsgemisch nach Anspruch 1, Dadurch gekennzeichnet, daß der Lipidanteil
1) Eicosapentaensäure C20 :5 (n – 3) (0,25 bis 0,75% des Lipidanteils) enthält und
2) folgenden Kriterien entspricht

$$\frac{\Sigma FS \text{ essentielle Fettsäuren}}{\Sigma FS \text{ gesättigte Fettsäuren}} := 0,4 \text{ bis } 1,0$$

$$\frac{\Sigma FS \text{ Fettsäuren Serie } (n - 6)}{\Sigma FS \text{ Fettsäuren Serie } (n - 3)} = 3,5 \text{ bis } 10,0$$

3. Nahrungsgemisch nach einem der Ansprüche 1 und 2, dadurch gekennzeichnet, daß es Chrom in der Kombinationsform Niacin (2 bis 8 mg/100 ml) und Chrom (10 bis 16,7 µg/100 ml) enthält.

4. Nahrungsgemisch nach einem der Ansprüche 1 bis 3, dadurch gekennzeichnet, daß es eine mit Arginin (0,66 g bis 1,07 g/100 ml) angereicherte Proteinquelle enthält.

## Claims

1. Polymeric complete nutrient mixture, in the form of a fine emulsion, intended for the oral or enteral feeding of the elderly, the said mixture containing proteins, lipids, carbohydrates, vitamins, minerals and trace elements, which mixture is characterised in that it contains
• calcium (> 0,066 g/100 ml) combined with fluorine (0.05 to 0.25 mg/100 ml) and with vitamin D (60 to 110 IU/100 ml),
• folic acid (0.13 to to (sic) 0.4 mg/100 ml), and
• zinc (2 to 4.0 mg/100 ml).

2. Nutrient mixture according to Claim 1, characterised in that the lipid intake provided
1) contains eicosapentaenoic acid C20 :5, (n – 3) (0.25 to 0.75% of lipid intake), and
2) fulfils the following criteria

$$\frac{\Sigma FA \text{ Essential fatty acids}}{\Sigma FA \text{ Saturated fatty acids}} = 0.4 \text{ to } 1.0$$

$$\frac{\Sigma FA \text{ } (n - 6) \text{ series fatty acids}}{\Sigma FA \text{ } (n - 3) \text{ series fatty acids}} = 3.5 \text{ to } 10.0$$

3. Nutrient mixture according to either of Claims 1 and 2, characterised in that it contains chromium in the form of a combination of niacin (2-8 mg/100 ml) and chromium (10 to 16-7 µg/100 ml).

4. Nutrient mixture according to any one of Claims 1 to 3, characterised in that it contains a protein source enriched in arginine (0.66 g to 1.07 g/100 ml).